# EUROPEAN PATENT SPECIFICATION

(11) **EP 3 033 122 B1**
(45) Date of publication and mention of the grant of the patent: **18.07.2018**
(21) Application number: 14802727.9
(22) Date of filing: 04.08.2014
(51) Int. Cl.: A61M 1/36, A61M 1/10, A61F 2/06, A61B 17/11

(54) **SYSTEMS FOR A FLUID CARRYING CONDUIT OF A VASCULAR ACCESS SYSTEM**
SYSTEME FÜR EINE FLÜSSIGKEITSLEITUNG EINES GEFÄSSZUGANGSSYSTEMS
SYSTÈMES CONCERNANT UN CONDUIT POUR TRANSPORT DE FLUIDE D'UN SYSTÈME D'ACCÈS VASCULAIRE

(30) Priority: 13.08.2013 US 201361865277 P; 18.09.2013 US 201361879462 P
(43) Date of publication of application: 22.06.2016
(73) Proprietor: Merit Medical Systems, Inc., South Jordan, UT 84095 (US)
(72) Inventor: TOMKO, Daniel, Dallas, Georgia 30132 (US); GALE, David, Kennesaw, Georgia 30152 (US); ARNOLD, Stacy, Cartersville, Georgia 30121 (US); KATRAGADDA, Venkata, Kennesaw, Georgia 30144 (US)
(74) Representative: Bradley, Adrian
(86) International application number: PCT/US2014/049547
(87) International publication number: WO 2015/023460

(56) References cited:
- EP-A2- 1 797 831
- WO-A1-01/05447
- WO-A1-90/08509
- WO-A1-2010/059102
- WO-A2-2009/059371
- WO-A2-2011/153302
- US-A- 3 818 511
- US-A- 3 853 126
- US-A1- 2012 059 305

## Description

### FIELD

The present disclosure relates generally to systems and methods for connecting multiple portions of a fluid carrying conduit, such as to provide a unitary fluid carrying conduit, in a vascular access system.

### BACKGROUND

In the United States, approximately 400,000 people have end-stage renal disease requiring chronic hemodialysis. Permanent vascular access sites for performing hemodialysis may be formed by creating an arteriovenous (AV) anastomosis, whereby a vein is attached to an artery to form a high-flow shunt or fistula. A vein may be directly attached to an artery, but it may take 6 to 8 weeks before the venous section of the fistula has sufficiently matured to provide adequate blood flow for use with hemodialysis. Moreover, a direct anastomosis may not be feasible in all patients due to anatomical considerations.

Other patients may require the use of artificial graft material to provide an access site between the arterial and venous vascular systems. Patency rates of grafts are still not satisfactory, as the overall graft failure rate remains high. Temporary catheter access is also an option. However, the use of temporary catheter access exposes the patient to additional risk of bleeding and infection, as well as discomfort.

Vascular access systems are known in the art. For example, U.S. Patent 6, 102,884 to Squitieri, U.S. Patent 7,762,977 to Porter, and U.S. Patent 8,079,973 to Herrig describe implantable blood conduit systems which include (i) an extravascular blood conduit that has a proximal end adapted to couple with a first vascular segment of a patient and a distal end adapted to be inserted into a second vascular segment of the patient; (ii) a catheter having a proximal portion and a distal portion that, when implanted, floats freely within the second vascular segment; and (iii) a connector for fluidly coupling the proximal end of the blood conduit with the catheter with the proximal portion. The interconnection of these various components, however, may cause turbulent flow between the vascular segments in some embodiments or circumstances. It therefore would be desirable to provide improved and/or alternative systems and methods for a blood conduit that eliminates and/or reduces turbulent flow through the vascular access systems. Moreover, it would be desirable to provide improved and/or alternative systems and methods for connecting multiple portions of a fluid carrying conduit that can readily be used with a variety of conduits or catheters, provide secure and reliable connections, and that physicians find relatively simple to use during an implantation procedure.

The document WO2010/059102 A1 discloses an anastomosis device including a graft vessel having a tubular portion with two open ends.

### SUMMARY

Some or all of the above needs and/or problems may be addressed by certain embodiments disclosed herein. The invention provides a system for fluidly coupling proximal and distal tubular segments of a fluid conduit in a cardiovascular system. In this manner, continuous flow may be provided between the proximal and distal tubular segments of the fluid conduit.

Also provided is a blood conduit for fluidly coupling a first vascular segment to a second vascular segment. The blood conduit includes a first conduit configured to be attached to the first vascular segment and a second conduit configured to be attached to the second vascular segment. The first conduit and the second conduit, as a unitary structure, collectively form a single unitary lumen. In this manner, smooth continuous flow may be provided through the lumen between the first vascular segment and the second vascular segment, as the absence of a connection/joint between the conduits provides a smooth sidewall for reducing or eliminating turbulence in the flow of blood through the conduits.

Other embodiments, aspects, and features of the disclosure will become apparent to those skilled in the art from the following detailed description, the accompanying drawings, and the appended claims.

### BRIEF DESCRIPTION OF THE DRAWINGS

The structure and method of using the devices, systems, and methods described herein will be better understood with the following detailed description of embodiments, along with the accompanying illustrations, which are not necessarily drawn to scale.
FIG. 1 is a perspective view of an embodiment of a vascular access system.
FIG. 2 is a perspective view of an embodiment of a system for connecting multiple portions of a fluid carrying conduit of a vascular access system.
FIG. 3 is a perspective view of an embodiment of a system for connecting multiple portions of a fluid carrying conduit of a vascular access system.
FIG. 4 is a perspective view of an embodiment of a system for connecting multiple portions of a fluid carrying conduit of a vascular access system.
FIG. 5 is a plan view of a system for connecting multiple portions of a fluid carrying conduit of a vascular access system.
FIG. 6 is a plan view of a system for connecting multiple portions of a fluid carrying conduit of a vascular access system.
FIG. 7 schematically depicts a perspective view of an embodiment of a vascular access system.

### DETAILED DESCRIPTION

Improved hemodialysis and vascular access systems and methods have been developed. Provided herein are systems and methods for connecting multiple portions of a fluid carrying conduit. For example, a blood conduit may include an inflow component (such as a proximal tubular segment) and an outflow component (such as a distal tubular segment). A first engagement feature is disposed on a distal end of the inflow component, and a second engagement feature is disposed on a proximal end of the outflow component. The first engagement feature is configured to mate directly with the second engagement feature. The inflow component and the outflow component are configured to mate by way of the first engagement feature and the second engagement feature without the use of an intermediate connector component. That is, in some instances, the systems and methods described herein do not include a connector component disposed between the inflow component and the outflow component. Instead, the first engagement feature is configured to mate directly with the second engagement feature. Secure connection of the inflow and outflow components by way of the first and second engagement features enables a continuous flow through the blood conduit. Techniques for connecting the inflow and outflow components are also provided. In various embodiments, the systems and methods described herein improve over, and/or build upon, the connecting means described in U.S. Patent No. 8,079,973, U.S. Patent No. 7,762,977, and U.S. Application Publication No. 2013/0060268.

Systems and methods are provided to enable fluid connection of the outflow component with the inflow component. The inflow component can be any of a variety of blood conduits that are able to be connected to the vascular system to receive blood into the vascular access system. Such blood conduits can have a construction similar to a vascular graft made of ePTFE, Dacron, or other suitable materials. Other suitable materials can include a material that is biocompatible with an artery and has a non- or minimally-thrombogenic characteristic. The inflow component preferably is adapted for long term attachment to an artery. The inflow component preferably includes a region suitable for repeated needle access. For example, a length of the inflow component can be configured to be pierced by a needle to enable blood to be withdrawn from and returned to the system, e.g., for dialysis.

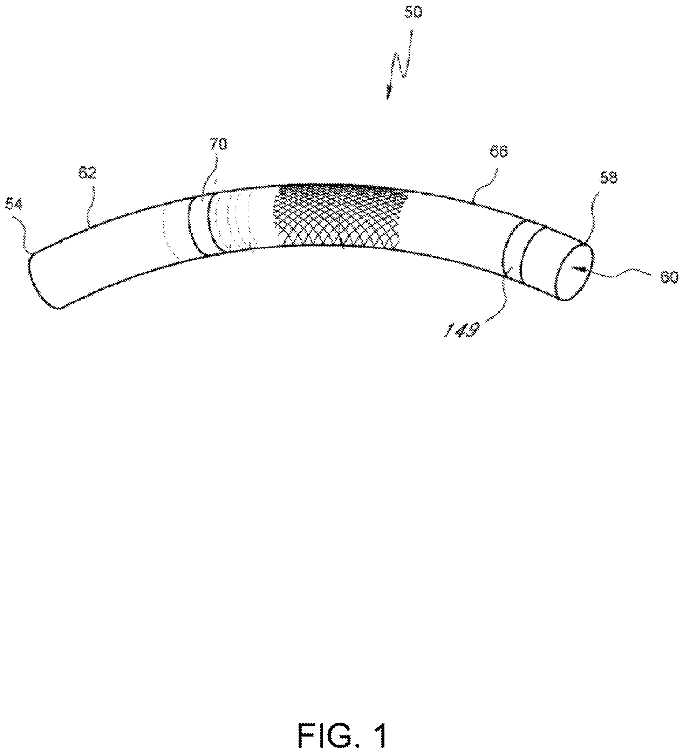

FIG. 1 illustrates one embodiment of a vascular access system 50 having a plurality of components that can be assembled together to form a lumen 60. The lumen 60 is configured to serve as a blood conduit or pathway configured to shunt blood from a first vascular segment to a second vascular segment. The vascular access system 50 has a proximal end 54 and a distal end 58. In some embodiments, the proximal end 54 can be adapted to couple with (e.g., attached to) a first vascular segment and the distal end 58 can be adapted to be coupled with (e.g., inserted into) a second vascular segment. The lumen 60 preferably extends between the proximal end 54 and the distal end 58. The lumen 60 can also be accessed from outside the patient to facilitate dialysis or other treatment.

In one embodiment, as illustrated in FIG. 1, the vascular access system includes a connector 70 adapted to fluidly connect a first conduit 62, such as an inflow component or graft, and a second conduit 66, such as an outflow component or catheter, to form the lumen 60. In certain embodiments, the first conduit 62 extends from the proximal end 54 toward the distal end 58, and the second conduit extends from the distal end 58 toward the proximal end 54. The connector 70 can be positioned between the first conduit 62 and the second conduit 66. In this manner, a distal portion of the first conduit 62 is configured to be connected to a proximal portion of connector 70, and a proximal portion of the second conduit 66 is configured to be connected to a distal portion of connector 70. The connector 70, first conduit 62, and/or second conduit 66 can be provided and/or integrated with one or more connecting devices to connect or enhance the security of connection between the first conduit 62 and the second conduit 66.

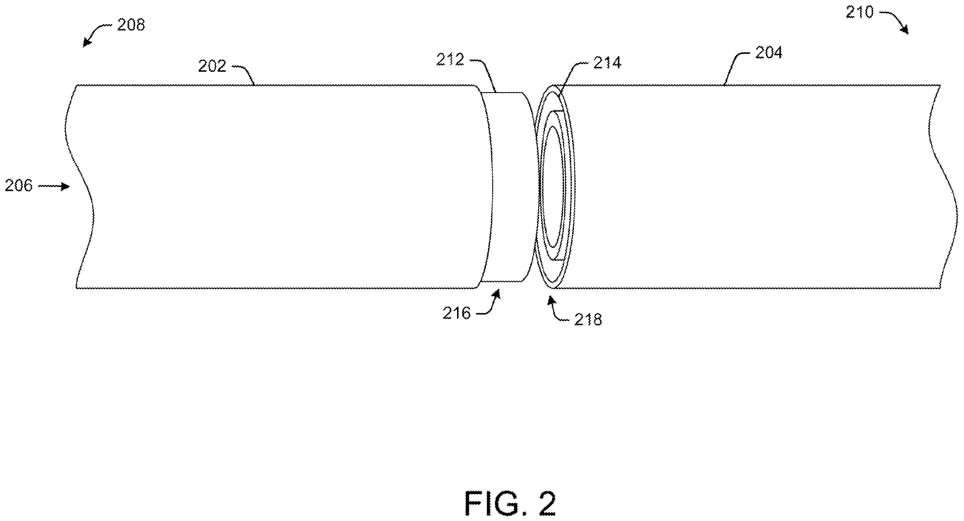

FIG. 2 illustrates one embodiment of a system 200 that can be incorporated into the blood conduit of the vascular access system 50 of FIG. 1. In certain embodiments, the first conduit 202 is be securely attached directly to the second conduit 204 without any intervening components. In this manner, the connector 70 of the vascular access system 50 of FIG. 1 may be omitted in the system 200.

When coupled together, the first conduit 202 and the second conduit 204 form a lumen 206. The lumen 206 provides a blood conduit or pathway configured to shunt blood from a first vascular segment to a second vascular segment. The lumen includes a proximal end 208 and a distal end 210. The lumen 206 extends between the proximal end 208 and the distal end 210. The proximal end 208 is adapted to couple with (e.g., attached to) a first vascular segment, and the distal end 210 is adapted to be coupled with (e.g., inserted into) a second vascular segment. The lumen 206 can also be accessed from outside the patient to facilitate dialysis or other treatment. In certain embodiments, the first conduit 202 may form an inflow component of the system 200, and the second conduit 204 may form an outflow component of the system 200. Further, in some instances, the first conduit 202 may comprise a proximal tubular segment, and the second conduit 204 may comprise a distal tubular segment.

In order to provide a connection between the first conduit 202 and the second conduit 202, the first conduit 202 includes a first engagement feature 212, and the second conduit 204 includes a second engagement feature 214. The first engagement feature 212 is disposed on a distal end 216 of the first conduit 202, and the second engagement feature 214 is disposed on a proximal end 218 of the second fluid conduit 204. The first engagement feature 212 is configured to mate with the second engagement feature 214 such that continuous flow is provided between the first conduit 202 and the second conduit 204 of the system 200. The lumens of the two components mate seamlessly so that the flow of blood through the lumen is smooth and unimpeded and the luminal interface of the two components provide no structures or surface sites for promoting thrombogenesis or damage to blood cells.

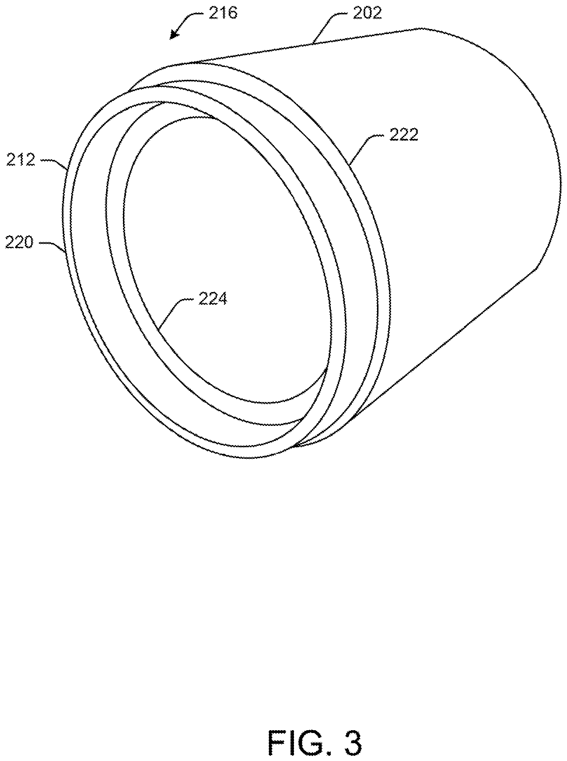

FIG. 3 depicts the first conduit 202. The first engagement feature 212 includes a protrusion 220 extending away from the distal end 216 of the first conduit 202. The protrusion 220 is attached to the distal end 216 of the first conduit 202 between an outer diameter 222 and inner diameter 224 of the first conduit 202. The protrusion 220 is integrally formed with and extends away from the distal end 216. The protrusion is located such that it forms an inner ledge or lip at the inner diameter 224 and an outer ledge or lip at the outer diameter 222, as shown in FIG. 3.

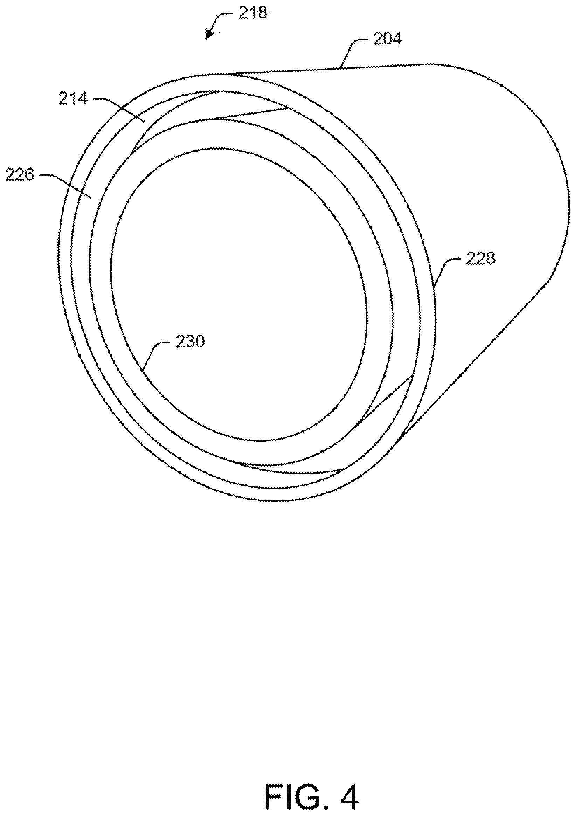

FIG. 4 depicts the second conduit 204. The second engagement feature 214 includes a groove 226 disposed within the proximal end 218 of the second conduit 204. The groove 226 is disposed between an outer diameter 228 and inner diameter 230 of the second conduit 204. The groove 226 is sized and shaped to receive the protrusion 220 therein. In this manner, the protrusion 220 and the groove 226 form a secure connection between the first conduit 202 and the second conduit 204. The inner diameter 224 of the first conduit 202 aligns with the inner diameter 230 of the second conduit 204 when the first conduit 202 and the second conduit 204 are secured together. Similarly, the outer diameter 222 of the first conduit 202 aligns with the outer diameter 228 of the second conduit 204 when the first conduit 202 and the second conduit 204 are secured together.

The protrusion 220 is secured within the groove 226 by frictional engagement of the components. The protrusion 220 and/or groove 226 includes a textured surface, an adhesive, a weld, or the like, to augment the securement. In other instances, the protrusion 220 may be press fit into the groove 226. In still other instances, external connection devices may be used in conjunction with the protrusion 220 and groove 226 connection. Any means may be used to secure the protrusion 220 within the groove 226.

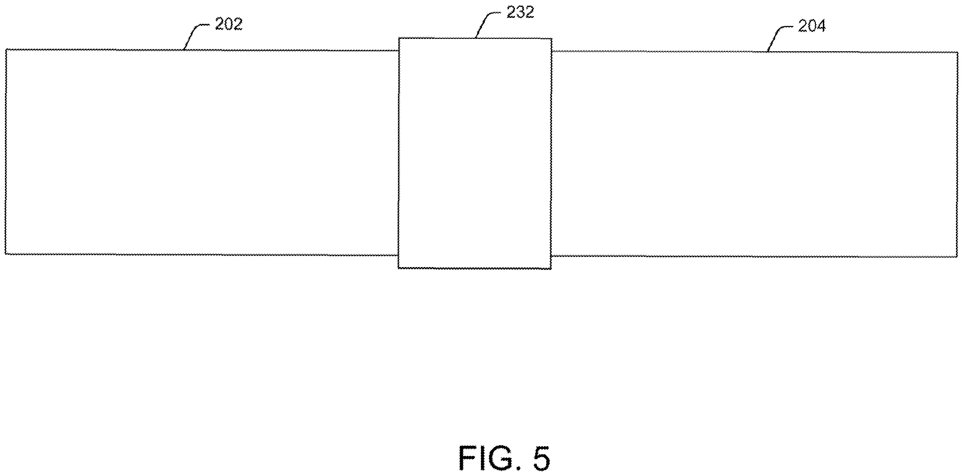

FIG. 5 depicts an external connection device 232. The external connection device 232 is positioned about the distal end 216 of the first conduit 202 and the proximal end 218 of the second conduit 204 when joined together. The external connection device 232 is configured to secure the distal end 216 of the first conduit 202 with the proximal end 218 of the second conduit 204. In some instances, the external connection device 232 may be used in conjunction with the first engagement feature 212 and/or the second engagement feature 214. In other instances, the external connection device 232 may be used independently of the first engagement feature 212 and/or the second engagement feature 214. The external connection device 232 may include an elastic band or sleeve, a clamp, a compression tube, and/or a combination thereof. Other external connection devices may also be used.

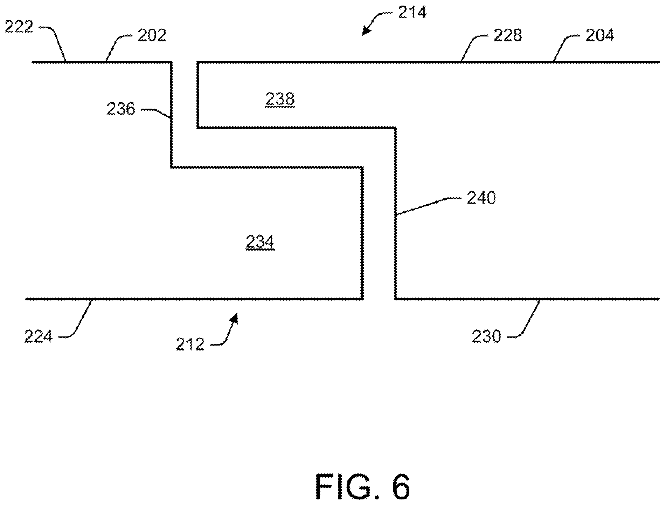

FIG. 6 depicts a first engagement feature 212 and a second engagement feature 214. The first engagement feature 212 is configured to mate with the second engagement feature 214 such that continuous flow is provided between the first conduit 202 and the second conduit 204. The lumens of the two components mate seamlessly so that the flow of blood through the lumen is smooth and unimpeded and the luminal interface of the two components provide no structures or surface sites for promoting thrombogenesis or damage to blood cells. The first engagement feature 212 comprises a protrusion 234 and a groove 236 defined by the inner diameter 224 and outer diameter 222 of the first conduit 202. Similarly, the second engagement feature 214 comprises a protrusion 238 and a groove 240 defined by the inner diameter 230 and outer diameter 228 of the second conduit 204. The protrusion 234 is configured to nest within the groove 240, and the protrusion 238 is configured to nest within the groove 236.

The various apparatuses, systems, and methods for connecting multiple portions of a fluid carrying conduit described in FIGS. 2-6 may be interchanged. That is, it is within the scope of the disclosure that certain of the connections described herein may include components, features, and/or functions of any of the other systems and methods disclosed. For example, the first conduit 202 may include the second engagement feature 214, and the second conduit 204 may include the first engagement feature 212. Moreover, the external connection device 232 may be used in conjunction with the first engagement feature 212 and/or the second engagement feature 214, while in other instances the external connection device 232 may be omitted.

The various apparatuses, systems, and methods for connecting multiple portions of a fluid carrying conduit described in FIGS. 2-6 may provide several technical advantages over the prior art. For example, the various engagement features of the conduits may facilitate ease of manufacturability of the vascular access system, ease of manufacturability of the graft assembly, greater security/performance of the connection, and/or provide the potential for easier disassembly in case of revision or replacement. Other advantages may become apparent throughout the disclosure.

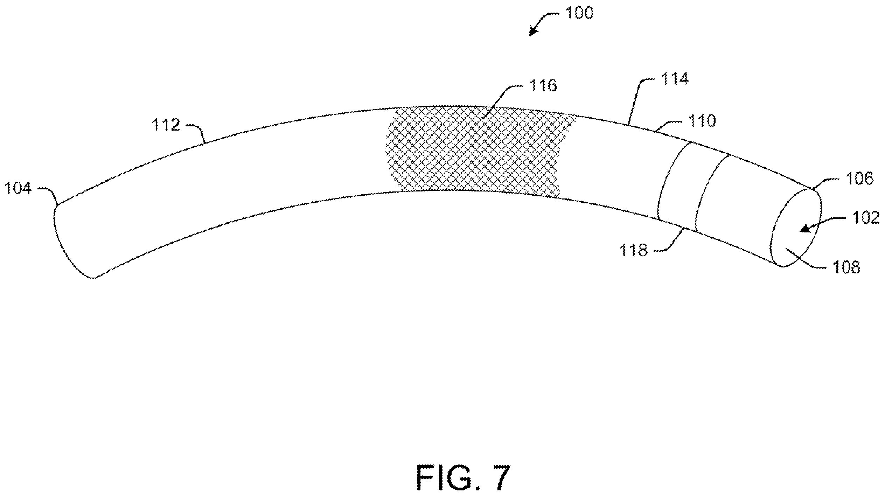

FIG. 7 depicts an embodiment for providing a unitary fluid carrying conduit, such as an implantable vascular conduit. The fluid carrying conduits may be arteriovenous (AV) shunts or catheters in various embodiments. For example, the embodiments described herein may improve flow between points in a patient's vasculature. In some instances, the vascular access system described herein may include a first portion of a blood-carrying conduit configured for use as an inflow component and a second portion of the blood-carrying conduit configured for use as an outflow component.

As noted above, the blood conduit includes an inflow component and an outflow component. The inflow component and the outflow component collectively form the blood conduit. In certain embodiments, the blood conduit comprises of a single unitary member. In this manner, a connection device between the inflow component and the outflow component advantageously may be omitted, forming a single unitary blood conduit between points in the patient's vasculature. In some instances, the inflow component may comprise the proximal end of the blood conduit, and the outflow component may comprise the distal end of the blood conduit. The blood conduit may be configured to provide continuous flow therethrough.

The systems and methods described herein may minimize and/or eliminate one or more connections between the various components of the blood conduit. As a result, turbulent blood flow through the blood conduit that would otherwise occur at a connection site between the inflow component and the outflow component may be reduced and/or eliminated. Further, because the blood conduit may comprise fewer components, manufacturing costs may be reduced. These and other advantages described herein may be useful in a number of environments that employ a vascular access system, such as vascular access devices, ventricular assist devices, total artificial hearts, and various types of hemodialysis systems. Other advantages may become apparent throughout the disclosure.

The inflow component can be any of a variety of blood conduits that are able to receive blood into the vascular access system from the patient's vasculature. Such blood conduits can have a construction similar to a vascular graft made of ePTFE (expanded polytetrafluoroethylene), Dacron, or other suitable materials. Any suitable graft material may be used. Other suitable materials can include a material that is biocompatible with an artery and is non- or minimally-thrombogenic. The inflow component may be adapted for attachment to an artery. For example, the inflow component may be attached to a brachial artery by an end-to-side anastomosis. In some instances, the inflow component preferably is adapted for long term attachment to an artery, although short-term applications may also be implemented.

The inflow component may include a region suitable for repeated needle access. For example, a length of the inflow component can be configured to be pierced by a needle to enable blood to be withdrawn from and returned to the system, e.g., for hemodialysis.

The outflow component can be any of a variety of blood conduits that are able to return blood from the vascular access system to a patient's vascular. In some instances, the outflow component may comprise a catheter for returning blood to the patient's vasculature. As used herein, "catheter" is a broad term that includes any blood carrying conduit that can be at least partially inserted into a blood vessel and advanced therein to a selected location, including into the atrium or elsewhere. In some instances, the outflow component may be attached by an end-to-side anastomosis to a vein. In other instances, the outflow component may extend into a vein or into the central venous system. The outflow component may be adapted such that at least a distal end portion thereof can freely float within a vascular segment when the vascular access system is applied to a patient. The inflow component and the outflow component may be made of the same material or different materials.

FIG. 7 illustrates one embodiment of a vascular access system 100 having a plurality of components that form a lumen 102. In embodiments, the lumen 102 is configured to serve as a blood conduit or pathway configured to shunt blood from a first vascular segment to a second vascular segment. The vascular access system 100 can take any suitable form, but preferably is adapted to be at least partially implanted beneath the skin of the patient. That is, the vascular access system 100 may be partially or completely implanted.

The vascular access system 100 has a proximal end 104 and a distal end 106. The proximal end 104 is adapted to couple with (e.g., attached to) a first vascular segment, and the distal end 106 is adapted to be coupled with (e.g., inserted into) a second vascular segment. The lumen 102 extends between the proximal end 104 and the distal end 106. The lumen 102 may be essentially any suitable length. In some instances, the length of the lumen 102 is adjustable. For example, at least a portion of the lumen 102 may be cut before, during, and/or after being inserted into the patient, which may be necessary to optimize the device with the particular anatomy (e.g., dimensions) of the patient.

The lumen 102 may be accessed from outside of the patient to facilitate dialysis or other treatment. The lumen 102 provides a pathway for blood to flow between the first vascular segment and the second vascular segment. The lumen 102 provides blood flow from an upstream to a downstream location without any periodic access required or anticipated. That is, the lumen 102 is configured to facilitate continuous flow therein.

The lumen 102 includes an inner wall 108 that in part defines the blood flow capacity of the lumen 102. The lumen also may include an outer wall 1 10 that in part defines the overall thickness of the lumen 102. The blood flow lumen 102 is substantially cylindrical, with the inner wall 108 defining a substantially circular cross-section. Similarly, the outer wall 110 defines a substantially circular cross-section. Lumens of other shapes and sizes can also be used herein. The outer and/or inner diameters of the lumen 102 can be substantially constant through the length of the lumen 102, or these may vary. The inner wall 108 is sufficiently smooth in surface finish to minimize turbulence. In some instances, the outer wall 110 is sufficiently smooth in surface finish to minimize trauma to the patient and to otherwise promote biocompatibility.

The vascular access system 100 includes a first conduit 1 12 (such as an inflow component or graft) and a second conduit 114 (such as an outflow component or catheter). The first conduit 112 and the second conduit 114 collectively form the lumen 102. The first conduit 112 comprises the proximal end 104 of the vascular access system 100, and the second conduit 114 comprises the distal end 106 of the vascular access system 100. The first conduit 112 includes a proximal tubular segment, and the second conduit 114 includes a distal tubular segment. The lumens of the first conduit 112 and the second conduit 114 interface seamlessly so that the flow of blood through the lumen 102 is smooth, unimpeded and void of any structures or surface sites for promoting thrombogenesis or damage to blood cells. In this manner, the first conduit 112 and the second conduit 114 are integrated to provide a single unitary member. That is, the first conduit 112 and the second conduit 114 may be a single piece, e.g., a monolithic structure.

The lumen of the first conduit 112 may be substantially cylindrical. Similarly, the lumen of the second conduit 114 may be substantially cylindrical. For example, the lumen of the first conduit 112 and the lumen of the second conduit 114 may be the same size and shape. In other instances, the lumen of the first conduit 112 and the lumen of the second conduit 114 may be different sizes and/or shapes. The lumen of the first conduit 112 and the lumen of the second conduit 114 may collectively form the lumen 102. The outer and inner diameter of the lumen of the first conduit 112 and the lumen of the second conduit 114 can be substantially constant through the length of the lumen 102 or can vary. The interface between the lumen of the first conduit 112 and the lumen of the second conduit 114 may be smooth and unimpeded and void of any structures or surface sites for eliminating or reducing turbulent flow therein. The interface between the first conduit 112 and the second conduit 114 does not have or make use of a connector device.

In some instances, the first and second vascular segments may be arterial or venous vascular segments. For example, the proximal end 104 can be adapted to be coupled with a brachial artery or other artery that resides close to the skin. Any suitable coupling between the proximal end 104 and the first vascular segment can be used. The proximal end 104 can be attached by an end-to-side anastomosis to a brachial artery. The distal end 106 can be adapted to couple with or extend into a vein, e.g., in the central venous system. Any suitable coupling between the distal end 106 and the second vascular segment can be used.

Although the first conduit 112 and the second conduit 114 may comprise a single unitary member, each may have different characteristics that are well suited for the unique ways in which the first conduit 112 and the second conduit 114 interact with the vasculature. For example, the first conduit 112 can be specifically configured to be integrated into the vessel with which it is coupled, e.g., by anastomosis connection to an artery. Also, the second conduit 114 can be specifically configured to interact with a vascular segment to minimize the likelihood of adverse side effects, e.g., by being flexible or otherwise formed to enable the distal portion 106 of the second conduit 114 to extend into the central venous system and interact in an atraumatic manner with vessel walls and other tissues in the vasculature or heart. Thus, the vascular access system 100 pertains to the unique requirements of a device that performs both as a permanently implanted extravascular graft and as an intravascular catheter.

The lumen 102 also can include a braided structure 116 or other reinforcing member disposed radially between the inner wall 108 and the outer wall 110 of the lumen 102. The braided structure 116 may be disposed within at least a portion of the first conduit 112, the second conduit 1 14, or a combination thereof. For example, the braided structure 116 may extend from the proximal end 104 to the distal end 106 of the lumen 102 or a portion therebetween. The braided structure 116 may be disposed between the inner wall 108 and the outer wall 110 of the lumen 102 such that the inner and outer surfaces of the lumen 102 are substantially smooth. The braided structure 116 may provide a number of benefits to the lumen 102. For example, the braided structure 116 can be configured to resist radial compression of the lumen 102. Also, the braided structure 1 16 can be configured to provide resistance to kinking of the lumen 102. Any suitable woven pattern can be provided for creating the braided structure 116. In some instances, the braided structure 116 may be omitted.

A radiopaque marker 118 may be disposed about one or more locations along the length of the lumen 102. For example, the radiopaque maker 118 may be disposed about the proximal end 104, the distal end 106, or somewhere therebetween. In some instances, the radiopaque marker 118 may comprise is a ring formed of platinum, tantalum, tungsten, gold, palladium, iridium, barium sulfate, or another radiopaque material, and/or any combination thereof. Although the marker 118 is configured as a solid ring, in other embodiments, these marking materials can be doped into layers of the lumen 102 or configured as a plurality of rings or one or more patches, plates, wires, beads, or other shapes. They may be distributed in the entire device or at one or both ends of the device or anywhere in between. In some instances, any other suitable device to provide the clinician with an indication of where the distal end 106 of the lumen 100 is located when the blood flow conduit is being advanced in the vasculature can be used instead of the radiopaque marker 118. In some instances, the radiopaque marker 118 may be omitted.

The various apparatuses, systems, and methods for providing a single unitary blood conduit may provide several technical advantages over the prior art. For example, the systems and methods described herein may minimize and/or eliminate one or more connections between the various components of the blood conduit, resulting in reduced or no turbulent blood flow through the blood conduit. Further, manufacturing costs may be reduced.

The foregoing vascular access systems depicted in FIGS. 2-7 can be embedded within a broader method of applying such a device to a patient. Such a method can involve accessing a vein in the patient into which an outflow component is to be inserted. For example, a jugular vein could be accessed in such a method. The distal end of the outflow component can be positioned distant from the access site, e.g., at any location between the access site to a chamber of the heart.

The proximal end of the outflow component can thereafter be positioned at any suitable anatomical location, e.g., at the nearest delta-pectoral groove. Such positioning of the proximal end of the outflow component can be achieved in any suitable manner, such as by tunneling subcutaneous ly the proximal end from adjacent to the venous insertion site to adjacent to the delta-pectoral groove.

The broader method can also include coupling the proximal end of an inflow component with a vascular segment different from the insertion site, which can be a jugular vein. The inflow component can be coupled with a different vascular segment, e.g., a brachial artery by any suitable technique. One technique involves suturing the proximal end to the artery, for example producing an end-to-side anastomosis. Attachment of the proximal end of the inflow component can be performed through a second incision formed through the skin adjacent to the vascular segment to which the inflow component is to be connected. After connecting the inflow component, the distal end of the inflow component can be tunneled, e.g., subcutaneously to an anatomical location suitable for coupling, in certain embodiments, to the outflow component, e.g., to the delta-pectoral groove, where a third incision can be formed.

Once distal and proximal portions of the inflow and outflow components respectively are located at a connection zone (e.g., delta-pectoral groove), connection of these components can be achieved using the systems and methods discussed above in FIGS. 2-6. In one convenient technique, a short length of a distal portion of the inflow component is lifted out of the patient through the third incision and a short length of a proximal portion of the outflow component is lifted out of the patient through the third incision. In some instances, the proximal end of the outflow component is attached to the distal end of the inflow component as discussed above in FIGS. 2-6. The inflow component and the outflow component may be a single unitary piece.

The foregoing devices and variants thereof enable the provision of a vascular access system. The inflow component can be attached by any means to an artery by suturing or otherwise arterialized. The proximal end of the inflow component may be attached by an expandable member, which can be self-expanding or balloon expandable. A self-expanding version can include a sinusoidal circumferential member adapted to be enlarged to at least the inner size of the artery. This enlargement enables a proximal portion of the inflow conduit to expand toward the inner wall of the artery, e.g., to be pressed into engagement with an internal segment of an artery. Another technique for arterializing the inflow component involves providing a coupling structure, which can be one or more stent-like structures, such as those described for example in U.S. Application Publication No. 2009/0076587. For example, at least a portion of the inflow component, e.g., including at least a portion of the coupling structure, can be deployed within the vessel and the remainder of the inflow component can extend from the vessel to the outflow component.

A portion of the outflow component may be adapted to be inserted within a vein at an insertion site. The outflow component can have an outside diameter which is less than an inner diameter of the vein and can have at least one opening in an end thereof with at least one of the openings in the catheter section to be disposed distant from the insertion site. For example, an outlet can be in the heart.

In operation, blood flows from the artery through the inflow component and is returned to the venous side of the circulatory system through an opening in the outflow component. The system preferably provides laminar blood flow between the artery and the vein. In certain applications, blood flows through the vein uninterrupted around at least an outer portion of the outflow component.

Access to the system can be provided in any suitable way, such as by providing a needle having a first end coupled to a hemodialysis device and having a second end adapted for insertion directly into the inflow component. Blood may thereby
be shunted from the vascular access device to a dialysis device and back to the patient's circulatory system.

Although disclosed in the context of certain preferred embodiments and examples, it will be understood by those skilled in the art that the present disclosures extend beyond the specifically disclosed embodiments to other alternative embodiments and/or uses of and obvious modifications and equivalents thereof. In addition, while several variations have been shown and described in detail, other modifications, which are within the scope of these disclosures, will be readily apparent to those of skill in the art. It is also contemplated that various combinations or sub-combinations of the specific features and aspects of the embodiments may be made and still fall within the scope of the disclosures. It should be understood that various features and aspects of the disclosed embodiments can be combined with or substituted for one another in order to form varying modes of the disclosed embodiments. Thus, it is intended that the scope of at least some of the present embodiments herein disclosed should not be limited by the particular disclosed embodiments described above.

## Claims

1. A system (50, 200) for fluidly coupling proximal (62, 202) and distal (66, 204) tubular segments of a fluid conduit in a cardiovascular system, the system comprising:
a first engagement feature (212) disposed on a distal end (216) of the proximal tubular segment; and
a second engagement feature (214) disposed on a proximal end (218) of the distal tubular segment, wherein the first engagement feature is configured to mate directly with the second engagement feature, whereby the proximal and distal tubular segments form a lumen and continuous flow is provided by the lumen between the proximal and distal tubular segments of the fluid conduit,
**characterized in that**
the first engagement feature comprises a circumferential groove disposed within the distal end of the proximal tubular segment, and
**in that** the circumferential groove is disposed between an outer and inner diameter of the proximal tubular segment.

2. The system of claim 1, wherein the first engagement feature further comprises a protrusion (220) extending away from the distal end of the proximal tubular segment.

3. The system of claim 2, wherein the protrusion is attached to the distal end of the proximal tubular segment between an outer (222) and inner (224) diameter of the proximal tubular segment.

4. The system of claim 1, wherein the second engagement feature comprises a groove (226) disposed within the proximal end (218) of the distal tubular segment.

5. The system of claim 4, wherein the groove is disposed between an outer (228) and inner (230) diameter of the distal tubular segment.

6. The system of claim 1, wherein the second engagement feature comprises a protrusion extending away from the proximal end of the distal tubular segment.

7. The system of claim 6, wherein the protrusion is attached to the proximal end of the distal tubular segment between an outer and inner diameter of the distal tubular segment.

8. The system of claim 1, wherein the second engagement feature comprises a protrusion and a groove defined by an inner diameter and an outer diameter of the proximal end of the distal tubular segment.

9. The system of claim 1, further comprising an external connection device (232) positioned about the distal end (216) of the proximal tubular segment (202) and the proximal end (218) of the distal tubular segment (204) when joined together, wherein the external connection device is configured to secure the distal end of the proximal tubular segment to the proximal end of the distal tubular segment.

10. The system of claim 9, wherein the external connection device comprises at least one of a clamp, a compression tube, or a combination thereof.

## Patentansprüche

1. System (50, 200) zum fluidischen Koppeln eines proximalen (62, 202) und eines distalen (66, 204) röhrenförmigen Segments einer Fluidleitung in einem Herzkreislaufsystem, wobei das System Folgendes umfasst:
ein erstes Eingriffsmerkmal (212), das an einem distalen Ende (216) des proximalen röhrenförmigen Segments angeordnet ist; und
ein zweites Eingriffsmerkmal (214), das an einem proximalen Ende (218) des distalen röhrenförmigen Segments angeordnet ist, wobei das erste Eingriffsmerkmal dazu konfiguriert ist, direkt mit dem zweiten Eingriffsmerkmal zusammenzupassen, wodurch das proximale und das distale röhrenförmige Segment ein Lumen bilden und von dem Lumen kontinuierlicher Durchfluss zwischen dem proximalen und dem distalen röhrenförmigen Segment der Fluidleitung bereitgestellt wird,
**dadurch gekennzeichnet, dass**
das erste Eingriffsmerkmal eine Umfangsnut umfasst, die in dem distalen Ende des proximalen röhrenförmigen Segments angeordnet ist, und
dadurch, dass die Umfangsnut zwischen einem Außen- und einem Innendurchmesser des proximalen röhrenförmigen Segments angeordnet ist.

2. System nach Anspruch 1, wobei das erste Eingriffsmerkmal weiter einen Vorsprung (220) umfasst, der sich von dem distalen Ende des proximalen röhrenförmigen Segments weg erstreckt.

3. System nach Anspruch 2, wobei der Vorsprung zwischen einen Außendurchmesser (222) und einem Innendurchmesser (224) des proximalen röhrenförmigen Segments an dem distalen Ende des proximalen röhrenförmigen Segments angebracht ist.

4. System nach Anspruch 1, wobei das zweite Eingriffsmerkmal eine Nut (226) umfasst, die in dem proximalen Ende (218) des distalen röhrenförmigen Segments angeordnet ist.

5. System nach Anspruch 4, wobei die Nut zwischen einem Außendurchmesser (228) und einem Innendurchmesser (230) des distalen röhrenförmigen Segments angeordnet ist.

6. System nach Anspruch 1, wobei das zweite Eingriffsmerkmal einen Vorsprung umfasst, der sich von dem proximalen Ende des distalen röhrenförmigen Segments weg erstreckt.

7. System nach Anspruch 6, wobei der Vorsprung zwischen einen Außen- und einem Innendurchmesser des distalen röhrenförmigen Segments an dem proximalen Ende des distalen röhrenförmigen Segments angebracht ist.

8. System nach Anspruch 1, wobei das zweite Eingriffsmerkmal einen Vorsprung und eine Nut umfasst, die von einem Innendurchmesser und einen Außendurchmesser des proximalen Endes des distalen röhrenförmigen Segments definiert werden.

9. System nach Anspruch 1, weiter umfassend eine externe Verbindungsvorrichtung (232), die um das distale Ende (216) des proximalen röhrenförmigen Segments (202) und das proximale Ende (218) des distalen röhrenförmigen Segments (204) positioniert ist, wenn diese zusammengefügt sind, wobei die externe Verbindungsvorrichtung dazu konfiguriert ist, das distale Ende des proximalen röhrenförmigen Segments an dem proximalen Ende des distalen röhrenförmigen Segments zu fixieren.

10. System nach Anspruch 9, wobei die externe Verbindungsvorrichtung mindestens eines aus einer Klemme, einem Kompressionsschlauch oder einer Kombination derselben umfasst.

## Revendications

1. Système (50, 200) pour le raccordement fluidique de segments tubulaires proximal (62, 202) et distal (66, 204) d'un conduit de fluide dans un système cardiovasculaire, le système comprenant :
un premier élément de prise (212) disposé sur une extrémité distale (216) du segment tubulaire proximal ; et
un deuxième élément de prise (214) disposé sur une extrémité proximale (218) du segment tubulaire distal, dans lequel le premier élément de prise est configuré pour s'accoupler directement avec le deuxième élément de prise, par quoi les segments tubulaires proximal et distal forment une lumière et l'écoulement continu est fourni par la lumière entre les segments tubulaires proximal et distal du conduit de fluide,
**caractérisé en ce que**
le premier élément de prise comprend une rainure circonférentielle disposée au sein de l'extrémité distale du segment tubulaire proximal, et
**en ce que** la rainure circonférentielle est disposée entre un diamètre externe et un diamètre interne du segment tubulaire proximal.

2. Système selon la revendication 1, dans lequel le premier élément de prise comprend en outre une saillie (220) s'étendant à distance de l'extrémité distale du segment tubulaire proximal.

3. Système selon la revendication 2, dans lequel la saillie est attachée à l'extrémité distale du segment tubulaire proximal entre un diamètre externe (222) et un diamètre interne (224) du segment tubulaire proximal.

4. Système selon la revendication 1, dans lequel le deuxième élément de prise comprend une rainure (226) disposée au sein de l'extrémité proximale (218) du segment tubulaire distal.

5. Système selon la revendication 4, dans lequel la rainure est disposée entre un diamètre externe (228) et un diamètre interne (230) du segment tubulaire distal.

6. Système selon la revendication 1, dans lequel le deuxième élément de prise comprend une saillie s'étendant à distance de l'extrémité proximale du segment tubulaire distal.

7. Système selon la revendication 6, dans lequel la saillie est attachée à l'extrémité proximale du segment tubulaire distal entre un diamètre externe et un diamètre interne du segment tubulaire distal.

8. Système selon la revendication 1, dans lequel le deuxième élément de prise comprend une saillie et une rainure définies par un diamètre interne et un diamètre externe de l'extrémité proximale du segment tubulaire distal.

9. Système selon la revendication 1, comprenant en outre un dispositif de connexion externe (232) positionné autour de l'extrémité distale (216) du segment tubulaire proximal (202) et de l'extrémité proximale (218) du segment tubulaire distal (204) quand elles sont reliées ensemble, dans lequel le dispositif de connexion externe est configuré pour fixer l'extrémité distale du segment tubulaire proximal à l'extrémité proximale du segment tubulaire distal.

10. Système selon la revendication 9, dans lequel le dispositif de connexion externe comprend au moins un élément parmi une pince, un tube de compression, ou une combinaison de ceux-ci.
